# EUROPEAN PATENT APPLICATION

(11) **EP 1 584 333 A2**
(43) Date of publication of application: **12.10.2005**
(21) Application number: 05015384.0
(22) Date of filing: 02.12.1998
(51) Int. Cl.: A61K 31/18

(54) **Use of acetylcoenzime A inhibitors for treating Alzheimer's disease**

(30) Priority: 28.01.1998 US 72912 P
(62) Divisional of application: 98960605.8
(71) Applicant: Warner-Lambert Company LLC, New Jersey 07950 (US)
(72) Inventor: Bisgaier, Charles Larry, Ann Arbor Michigan 48105 (US); Emmerling, Mark Richard, Chelsea Michigan 48118 (US)
(74) Representative: Krohn, Sabine Martha Erna

(57) **Abstract**

The present invention provides a method for treating or preventing the onset of Alzheimer's Disease comprising administering to a mammal in need thereof an Alzheimer's Disease-preventing or treating amount of a plasma-triglyceride level-lowering agent. Optionally, the plasma-triglyceride level-lowering agent can be co-administered with a cholesterol level-lowering agent.

## Description

### BACKGROUND OF THE INVENTION

The present invention relates to the field to therapeutic treatments of Alzheimer's disease.

### SUMMARY OF THE RELATED ART

Alzheimer's Disease (AD) is characterized by the accumulation of insoluble, 10 nm filaments containing β-amyloid (Aβ) peptides, localized in the extracellular space of the cerebral cortex and vascular walls. These 40 or 42 amino acid long Aβ peptides are derived from the larger β-amyloid precursor protein (βAPP) through the endopeptidase action of β and γ secretases. In addition, the post-translational action of putative aminopeptidases results in a heterogeneous shortening of the 40 or 42 amino acid long Aβ peptides that either terminate at residue 40 or 42 and, therefore, are designated as Aβ N-40 and Aβ N-42. In familial forms of AD, the pathological appearance of the Aβ peptides in the brain is driven by the presence of mutations in the βAPP gene or in the genes coding for the proteins presenilin 1 and 2.

Sporadic AD accounts for more than 95% of the known AD cases. Its etiology, however, remains obscure. An accepted view is that sporadic AD results from the interplay between an individual's genetic factors and the environment, leading to the deposition of Aβ, neurodegeneration, and dementia. Despite this emerging perspective, few efforts have been made in identifying factors responsible for Aβ accumulation in the brain.

Epidemiological investigations clearly indicate that cardiovascular diseases increase the risk of developing AD. Several studies have also demonstrated a high incidence of often neglected cardiovascular problems in the AD population. Moreover, those with cardiovascular disease, but no overt dementia, frequently exhibit AD-like neuropathological lesions in their brains.

Several lines of evidence suggest that cholesterol and cholesterol metabolism might influence susceptibility to AD. Two previous clinical studies showed that total serum or LDL cholesterol was elevated in patients with AD. Moreover, individuals who are ApoE ε4, a well recognized risk factor for cardiovascular disease and AD, also tend to manifest hypercholesterolemia. In addition, the incidence of AD appears to be higher in countries with high fat and high caloric diets, and decreased in populations ingesting diets that decrease cardiovascular disease. Epidemiological investigations have further demonstrated that the risk for AD was greater in individuals with high cholesterol levels, and that the onset of AD occurred earlier in those individuals who were ApoE ε4 with high serum cholesterol. It may also be significant that polymorphic variations in genes coding for the lipoprotein-like receptor protein (LRP) and apolipoprotein ApoE4 might increase susceptibility to AD.

WO 95/06470 discloses methods for treating, arresting the development of, and preventing Alzheimer's disease by regulating the amount of ApoE isoform 4 circulating in the bloodstream and in the brain, comprising employing an HMG-CoA reductase inhibitor, e.g., lovastatin, simvastatin, pravastatin, and fluvastatin.

WO 97/48701 discloses 4,1-benzoxazepines and 4,1-benzothiazepines as squalene synthase inhibitors and propose their use as anti-AD agents.

Studies have shown that pharmaceutical reduction of blood cholesterol with statins or bile sequestrants reduce vascular and cardiac disease. Similarly, high blood triglyceride levels are also associated with certain types of vascular and cardiac diseases ("VCD"). It has heretofore been unknown, however, whether reduction of plasma triglycerides delays onset of AD.

### SUMMARY OF THE INVENTION

The present invention comprises a new method for treating and preventing the onset of Alzheimer's Disease. In one aspect, a method of treating AD is provided, the method comprising administering to a mammal suffering from AD an AD-alleviating amount of an agent that lowers the mammal's blood triglyceride level or otherwise regulates lipids. In another aspect, a method of preventing the onset of AD is provided, the method comprising administering to a mammal an AD-preventing amount of an agent that lowers the mammal's blood triglyceride level.

In another aspect of the invention, methods of treating and preventing AD are provided, which methods comprise administering to a mammal a combination of agents that lower the mammal's blood triglyceride level and its cholesterol level.

In another aspect of the invention, methods of treating and preventing AD are provided, which methods comprise administering to a mammal a combination of agents that lower the mammal's blood triglyceride level and its LDL-cholesterol (LDL-C) level and raise its HDL level.

In yet another aspect of the invention, methods of treating and preventing AD are provided, which methods comprise administering to a mammal an agent that raises the mammal's HDL cholesterol level. In another aspect, the HDL cholesterol (HDL-C) level-raising agent is administered in combination with an LDL-C cholesterol lowering agent.

The foregoing merely summarizes certain aspects of the invention and is not intended, nor should it be construed, as limiting the invention in any manner. All patents and other publications cited herein are hereby incorporated by reference in their entirety.

### BRIEF DESCRIPTION OF FIGURES

Figure 1 shows the differences in LDL cholesterol levels between AD patients and non-AD control patients (ND).
Figure 2 shows the comparison of brain grey matter cholesterol in AD patients and in non-AD patients (2A), and the comparison of brain white matter cholesterol in AD patients and in non-AD patients (2B).
Figure 3A shows the amount of insoluble fibrillar Aβ N-40 in brains of AD patients segregated by ApoE genotype (i.e., ε3,ε4), compared to non-AD controls (ND), also segregated by ApoE genotypes.
Figure 3B shows the amount of insoluble fibrillar Aβ N-42 in brains of AD patients, compared to non-AD controls (ND).
Figure 3C shows the amount of total fibrillar Aβ protein in brains of AD patients, compared to non-AD controls (ND).
Figure 4 shows the correlations between brain fibrillar Aβ N-42 and serum lipoproteins and ApoB. The relationships between brain Aβ N-42 with serum total cholesterol (4A), LDL cholesterol (4B), apolipoprotein B (4C) and HDL cholesterol (4D) in control (C) and AD subjects are shown.
Figure 5 shows the dose-dependent inhibition of β-amyloid protein in CHO cell culture caused by several statin cholesterol lowering agents, namely mevastatin, lovastatin, pravastatin, and simvastatin.
Figure 6 shows the activity of CI-1011 (avasimibe), CI-1027, CI-719 (gemfibrozil), and PD 69405 to reduce β-amyloid concentrations in CHO cells.

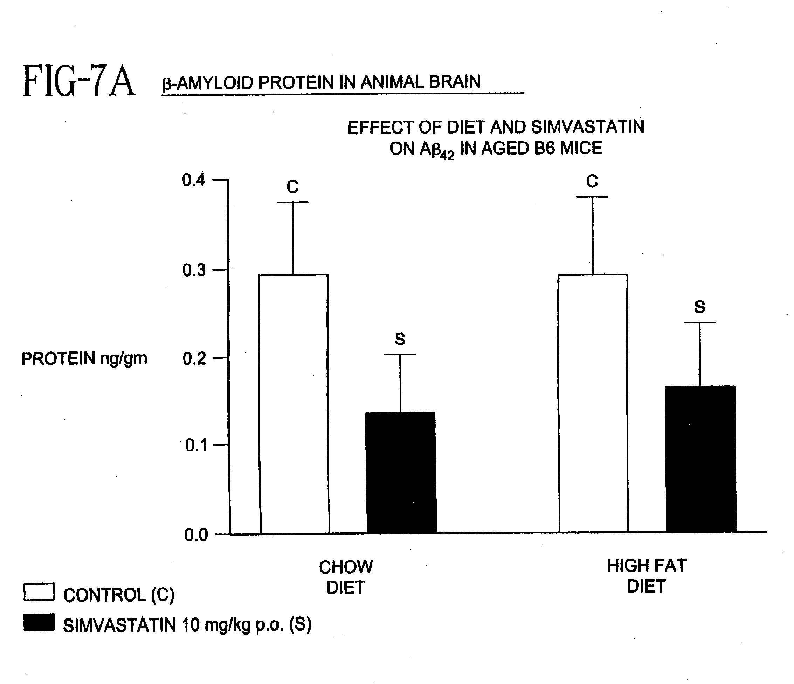

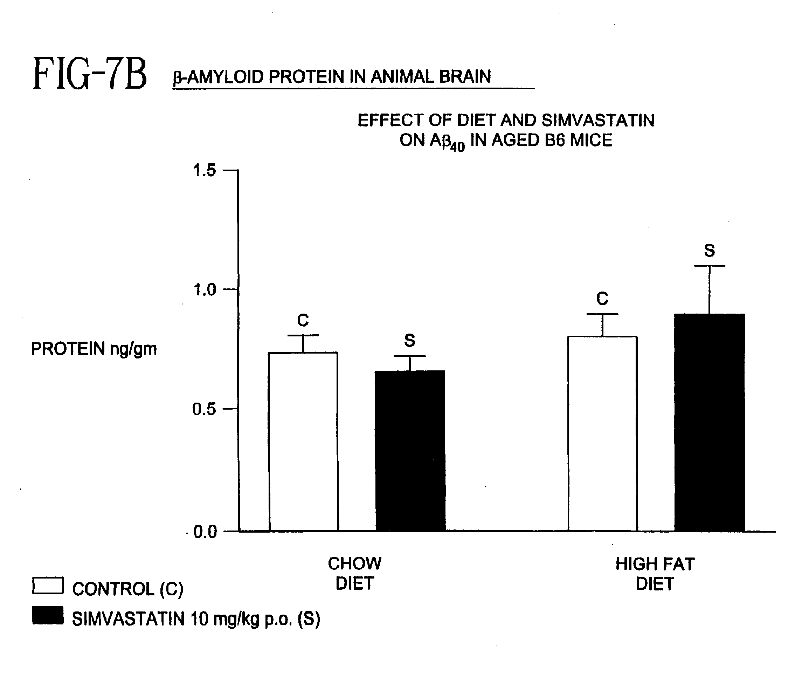

Figure 7 shows the effect on the concentration of β-amyloid protein N-42 and N-40 in animal brains following dosing with a lipid regulating agent (simvastatin, S) relative to controls (C).

### DETAILED DESCRIPTION OF THE PREFERRED EMBODIMENTS

The present invention comprises a new method for treating and preventing or delaying the onset of Alzheimer's Disease. It is born by the observation that risk factors for cardiovascular disease can have a profound impact on the expression of Aβ in AD brains. The data presented herein implicates ApoE ε4 status as the major determinant in the expression of Aβ N-40. Also, independent of ApoE genotype, higher levels of plasma cholesterol in the form of LDL are related to higher concentrations of Aβ N-42 in the AD brain. In addition, the data show a benefit of having an elevated ratio of HDL-C relative to very low density lipoprotein cholesterol (VLDL-C), plus low density lipoprotein cholesterol (LDL-C), in reduction of AD. The data clearly establishes the participation of plasma cholesterol in the pathophysiology of AD. Other studies have shown that other neurological disorders, such as vascular dementia and stroke, are related to hypercholesterolemia and hypertension. In these latter diseases, retrospective and prospective epidemiological studies have demonstrated that the use of antihypertensive agents or control of plasma cholesterol levels, through diet and drugs, have decreased the morbidity and mortality caused by these diseases. Thus, regulation of cardiovascular risk factors can also offer an as yet unexplored avenue to prevent or at least delay the occurrence of Alzheimer's Disease.

In view of the foregoing, therefore, in one aspect of the invention, a method of treating Alzheimer's Disease is provided, the method comprising administering to a mammal suffering from Alzheimer's Disease an Alzheimer's Disease-alleviating amount of a plasma triglyceride level-lowering agent. Numerous triglyceride level-lowering agents are known, and include, but are not limited to, fibrates (e.g., clofibrate, gemfibrozil (CI-719), fenofibrate, ciprofibrate, and bezafibrate), niacin, carboxyalkethers, thiazolinediones, eicosapentaenoic acid (EPA) and EPA-containing compositions (e.g., Max EPA, SuperEPA).

Thiazolinediones useful in the present invention include, for example, darglitazone, pioglitazone, BRL49653 (rosiglitazone), and troglitazone.

Carboxyalkylethers useful in the invention are described in U.S. Patent No. 5,648,387. Specifically, such compounds have the structure of Formula I 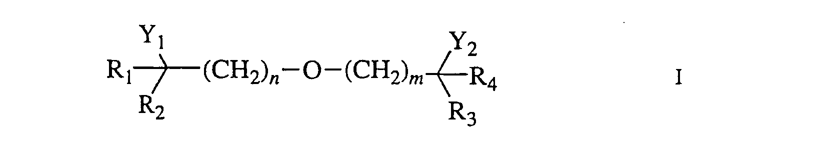 wherein
- n and m: independently are integers from 2 to 9;
- R₁, R₂, R₃, and R₄: independently are C₁-C₆ alkyl, C₂-C₆ alkenyl, C₂-C₆ alkynyl, and R₁ and R₂ together with the carbon to which they are attached, and R₃ and R₄ together with the carbon to which they are attached, can complete a carbocyclic ring having from 3 to 6 carbons;
- Y₁ and Y₂: independently are COOH, CHO, tetrazole, and COOR₅ where R₅ is C₁-C₆ alkyl, C₂-C₆ alkenyl, C₂-C₆ alkynyl;
and where the alkyl, alkenyl, and alkynyl groups may be substituted with one or two groups selected from halo, hydroxy, C₁-C₆ alkoxy, and phenyl.

Preferred carboxyalkylethers for use in the invention have the above formula wherein n and m are the same integer, and wherein R₁, R₂, R₃, and R₄ each are alkyl.

Further preferred carboxyalkylethers are those in which Y₁ and Y₂ independently are COOH or COOR₅ where R₅ is alkyl.

The most preferred carboxyalkylethers for use in the invention have the formula 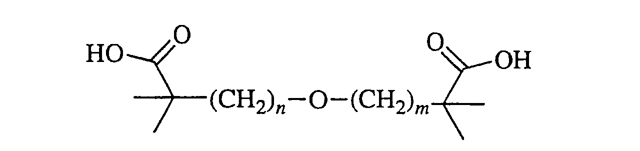 wherein n and m are each an integer selected from 2, 3, 4, or 5, ideally 4 or 5.

An especially preferred carboxyalkylether for use in the invention is CI-1027, which has the formula

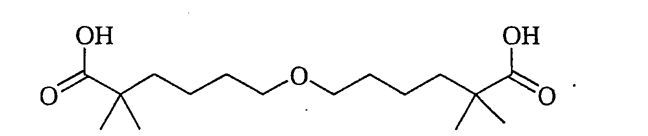

Another group of lipid regulators which lower triglycerides and which can be used according to this invention are inhibitors of acyl-coenzyme A:cholesterol acyltransferase (ACAT). Such ACAT inhibitors are well-known, for example, as described in U.S. Patent No. 5,491,172. These compounds have the general structure 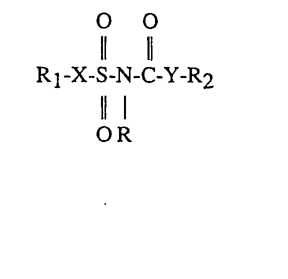 wherein X and Y are O, S, or (CR'R")ₙ, n is 1 to 4, R is hydrogen, alkyl or benzyl, R₁ and R₂ include aryl and cycloalkyl. One compound from within this group is especially preferred, namely 2,6-bis(1-methylethyl)phenyl[2,4,6-tris(1-methylethyl)phenyl]acetyl]sulfamate, now generically known as avasimibe, and also known as CI-1011.

Other commonly available plasma triglyceride-lowering agents can also be employed. One such compound is PD 69405, which a has the structure

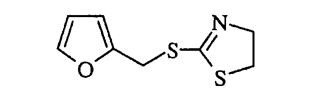

In another embodiment of this aspect of the invention, a method for treating AD is provided in which the plasma triglyceride level-lowering agent is co-administered with an effective plasma cholesterol lowering amount of a plasma cholesterol level-lowering agent. Many such plasma cholesterol-level-lowering agents useful in this embodiment are known and include, but are not limited to, statins (e.g., lovastatin (U.S. Patent No. 4,231,938), mevastatin (U.S. Patent No. 3,983,140), simvastatin (U.S. Patent No. 4,444,784), atorvastatin, cerivastatin (U.S. Patent No. 5,502,199 and EP 617019), velostatin (U.S. Patent Nos. 4,448,784 and 4,450,171), flurastatin (U.S. Patent No. 4,739,073), dalvastain (EP Appln. Publn. No. 738510 A2), fluindostatin (EP Appln. Publn. No. 363934 A1) and pravastatin (U.S. Patent No. 4,346,227), the bile acid sequestrants (e.g., cholestyramine and colestipol), and agents that block intestinal cholesterol absorption, e.g., β-sitosterol, SCH48461, CP-148,623 (Harris et al., *Clin. Pharm. Therap.,* 1997;**61**:385), saponins, neomycin, and ACAT (acyl-CoA:cholesterol acyltransferase) inhibitors. The patent art is rich with compounds that inhibit cholesterol biosynthesis, as evidenced by U.S. Patent Nos. 5,468,771, 5,447,717, 5,385,932, 5,376,383, 5,369,125, 5,362,752, 5,359,096, 5,326,783, 5,322,855, 5,317,031, 5,310,949, 5,302,604, 5,294,627, 5,286,895, 5,284,758, 5,283,256, and 5,278,320.

In a second aspect of the invention, a method of preventing the onset of Alzheimer's Disease is provided, the method comprising administering to a mammal an Alzheimer's Disease-preventing amount of a plasma triglyceride level-lowering agent. Such plasma triglyceride level-lowering agents are known in the art and include those recited above.

In another embodiment of this aspect of the invention, a method of preventing the onset of AD is provided in which the plasma triglyceride level-lowering agent is co-administered with an effective plasma cholesterol-lowering amount of a plasma cholesterol level-lowering agent. Many such plasma cholesterol-level-lowering agents are useful in this embodiment are known and include those recited previously.

In another aspect of the invention, methods of treating and preventing AD are provided, which methods comprise administering to a mammal a combination of agents that lower the mammal's blood triglyceride level and its LDL-cholesterol (LDL-C) level and raise its HDL level. Agents that reduce LDL-C levels are known and include HMG-CoA reductase (HMGR) inhibitors, especially the statins such as atorvastatin, lovastatin, simvastatin, pravastatin, rivastatin, mevastatin, fluindostatin, cerivastatin, velostatin, fluvastatin, dalvastain, as well as dihydrocompactin (U.S. Patent No. 4;450,171), compactin (U.S. Patent No. 4,804,770), and neomycin. Atorvastatin calcium is particularly preferred (U.S. Patent No. 5,273,995). HDL level-increasing drugs include gemfibrozil and simvastatin, and especially the carboxyalkylethers mentioned above, for example CI-1027.

In yet another aspect of the invention, methods of treating and preventing AD are provided, which methods comprise administering to a mammal an agent that raises the mammal's HDL cholesterol level. In another aspect the HDL cholesterol (HDL-C) level-raising agent is administered in combination with an LDL-C lowering agent.

Besides the agents expressly recited herein, there are many known agents useful in the various aspects of the invention, many of which are described in *The Merck Index* (Eleventh Edition) (Budavari et al., Eds., Merck & Co., Inc., Rahway, NJ) and the Physician's Desk Reference (Medical Economics Data Production Co., Montvale NJ). Pharmaceutically acceptable salts of the compounds useful in the invention can also be used. It will also be clear to those skilled in the art that more than one agent can be used for any particular purpose, as can pharmaceutically acceptable compositions comprising one or more agents.

The amounts of agents suitable for use in the various aspects of the invention are readily and routinely determinable by those skilled in the art using standard, art recognized methods. For example, to determine effective and optimal amounts of triglyceride level-lowering agents useful for treating AD, several groups of patients suffering from AD should be followed. One group, the control group, is to be administered a placebo. The remaining groups are administered varying amounts of a triglyceride level-lowering agent, and the cognitive skills of the individuals in each of the groups monitored to determine which group or groups manifest better cognitive skills compared to the control group. Similar routine studies can be conducted to determine effective and optimal amounts of such agents for preventing and/or delaying the onset of AD, with and without the co-administration of a cholesterol level-lowering agent.

In general, however, amounts of triglyceride level-lowering agent and cholesterol level-lowering agent useful in all aspects of the invention are those that are commonly and routinely used for the treatment of vascular and cardiac disease. Relatedly, regimes for administration of the agents for use in the treatment of vascular and cardiac disease can be used in the various aspects of the present invention. Such agents typically are administered at doses of about 0.1 mg to about 1000 mg per day, and ideally at about 5 mg to about 100 mg per day. The combinations to be employed can be formulated individually in their normal fashion (e.g., atorvastatin, troglitazone, rosiglitazone, gemfibrozil), or the agents can be formulated as a fixed dose combination, for example, an oral tablet containing 40 mg of atorvastatin and 200 mg of gemfibrozil or carboxyalkylether.

Administration of the agents recited in each aspect of the invention can be conducted by the same methods the agents are administered to treat vascular and cardiac disease, which are widely known and commonly used.

The ability of the triglyceride level-lowering agents and the cholesterol level-lowering agents to prevent or delay the onset of AD has been established by the following detailed examples. The examples are provided for illustrative purposes only, and are not intended to be limiting in any respect.

### EXAMPLE 1

Apolipoprotein E is a 34 kDa amphipathic protein that associates with serum triglyceride-rich and high-density lipoproteins and is involved in the transport of cholesterol between tissues. Three isoforms of the ApoE protein that differ by one or two amino acids are found in the human population. The ApoE2, E3, and E4 are respectively coded by the genes ApoE ε2, ε3, and ε4. Apoliprotein E ε4 represents a well-established risk factor for AD. Individuals with AD carrying the ApoE ε4 allele have more profuse deposits of Aβ in the cerebral cortex and vascular walls than the other ApoE alleles. This implies that ApoE4 interactions with Aβ or its lipid transport function or both affect the accumulation of Aβ. The increased risk of cardiovascular disease conferred by ApoE4 is attributed to an associated hypercholesterolemia that can promote or exacerbate atherosclerosis, hypertension, myocardial infarction and critical coronary artery disease.

The following experiment investigated the relationship between AD and known risk factors for cardiovascular disease, including ApoE genotype, serum lipids, lipoproteins, and apolipoprotein levels. In addition, Aβ levels in the gray matter were determined. The results are discussed in terms of the implicit involvement of lipid metabolism in the pathophysiology of Alzheimer's Disease.

### HUMAN SUBJECTS AND METHODOLOGY

*Human Tissue.* Sixty-four AD and 36 non-demented control brains were obtained from Sun Health Research Institute Brain Bank (postmortem-freezing delay 1-3 hours, average 2.1 hours). The brains from the demented patients fulfilled the diagnostic criteria of AD as dictated by the Consortium to Establish a Registry for Alzheime's Disease (CERAD). The control cases had no clinical history of dementia or neurological symptoms, and on neuropathological examination did not meet the AD guidelines. Blood was collected in the immediate post-mortem by cardiac puncture from left ventricle.

*ApoE genotyping*. ApoE genotyping was carried out using standard techniques. Crude genomic DNA, prepared from white blood cell nuclei, was submitted to 40 cycles of polymerase chain reaction, and digested with restriction enzyme *Hha*I prior to electrophoresis on an 8% polyacylamide gel.

*Quantitation of lipids.* Serum total cholesterol and triglycerides were determined enzymatically by standard procedures. Serum lipoprotein cholesterol profiles and distribution among lipoproteins were determined by on-line post column analysis on Superose 6HR high performance gel filtration chromatography (HPGC). Lipoprotein cholesterol was determined by multiplying the independently determined total serum cholesterol by the percent area for each lipoprotein distinctly separated by the HPGC method. ApoA-I, ApoE and ApoB levels were determined by immunoturbidometric methods using commercially available kits (Wako Chemical USA, Inc., Richmond, VA) on a Cobus Mira Plus analyzer (Roche Diagnostics Systems, Branhburg, NJ).

*Quantitation of brain cholesterol.* Brain lipids were extracted by standard methods. Briefly, 0.2 g of white or grey brain tissue, plus 100 µg of 4-cholesten-3-one (internal standard) was homogenized in 5 mL of chloroform/methanol (2:1, v/v) and then filtered through Whatman No. 1 filter paper. Another 2 mL of the chloroform/methanol mixture was used to re-extract the residue. Water (1.5 mL) was added to the extract and centrifuged at 2000 g for 10 minutes to distinctly separate the biphase. The lower chloroform phase containing the lipid extract was taken to dryness under nitrogen gas, and then dissolved in 1 mL of 2-propanol/hexane (1:19, v/v) for HPLC analysis. Brain cholesterol was separated by high pressure liquid chromatography (Thermo Separation Products, Freemont, CA) from internal standard on a 5 µm silica normal phase column (Zorbax SIL, 4.6 x 250 mm) at a flow rate of 1 mL/minute. The relative absorbance values at 208 nm for the internal standard and cholesterol were considered in the final calculation of brain cholesterol.

*Europium immunoassay (EIA) of Aβ Peptides*. Cerebral cortex (0.8 g) from the superior frontal gyrus was minced and rinsed with buffer (20 mM Tris-HCl, pH 8.5) containing protease inhibitors. The tissue was homogenized in 3 mL of buffer, spun at 100,000 g for 1 hour at 4°C and prepared for Aβ quantitation. One hundred microliters of the final diluted solution was submitted to EIA. Rabbit antibodies R163 and R165, raised against amino acids 34-40 and 36-42 of Aβ, respectively, were coated to microtiter plates. Wells were blocked with bovine serum albumin (1%) and 100 µL of the specimens or of Aβ standards were applied, incubated at room temperature for 2 hours, and then rinsed with 0.05% Tween 20-tris buffered saline (TTBS). Europium-labeled 4G8 antibody (against Aβ residue 17-24) was added to the wells, incubated for 2 hours and washed with TTBS, and rinsed with deionized water. Finally, the Eu enhancement solution (Wallac Inc., Gaithersburg, MD) was added and the plates read in a fluorimeter using excitation and emission wavelengths of 320 and 615 nm, respectively. The values, obtained from triplicated wells, were calculated based on standard curves generated on each plate.

*Statistical Analysis.* Two-tailed Student T-Test was applied when variable means were compared between control and AD subjects. Analysis of covariance (ANCOVA) of linear regression was used to estimate the relationships between two variables. The effects of ApoE genotype were determined by analysis of variance (ANOVA). Post-hoc multiple comparisons were only applied to those significant ANOVA groups. Significant differences between genotypes were determined by Fisher's Protected Least Significant Differences (PLSD) for the comparisons of multiple means.

### RESULTS

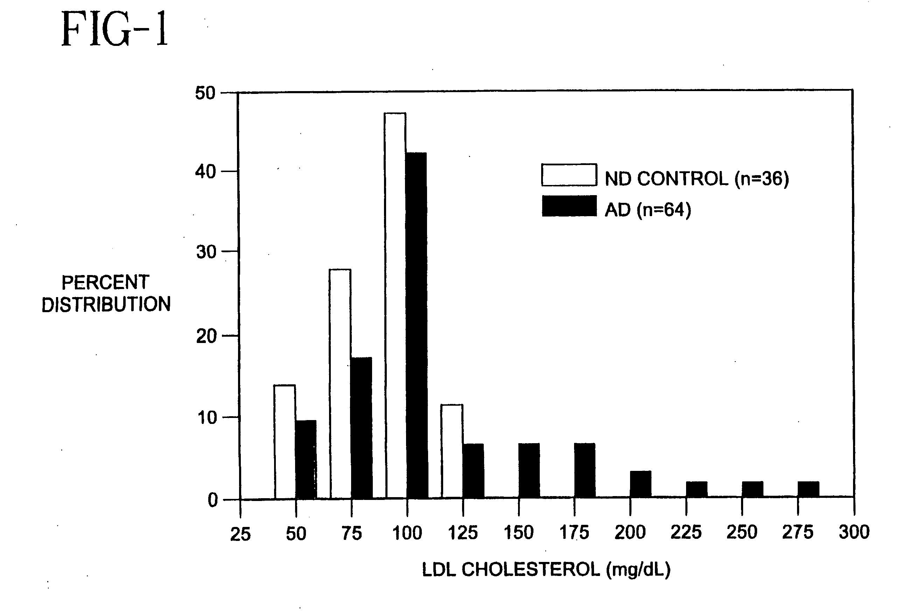

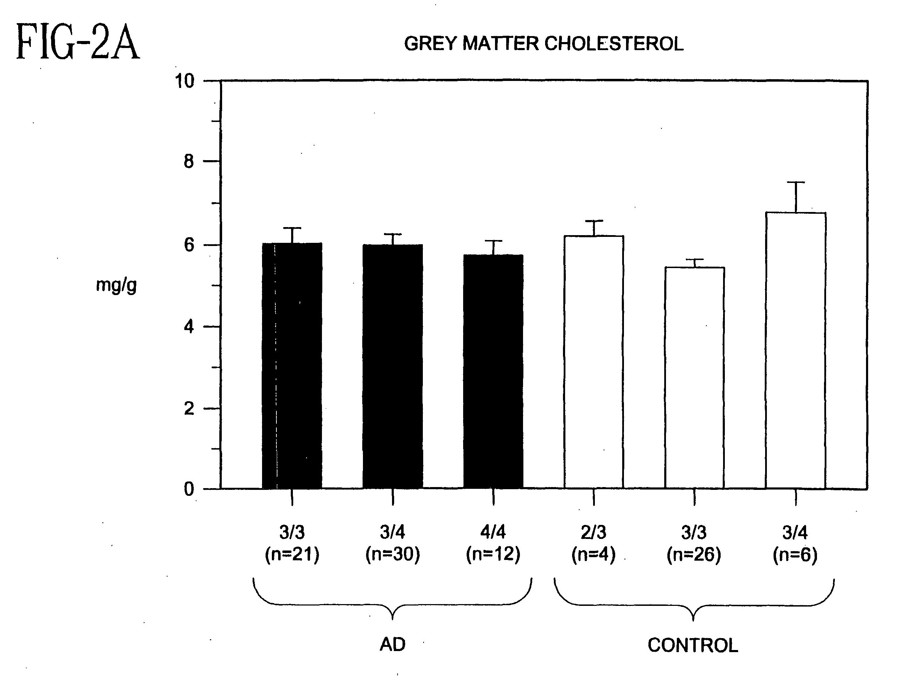

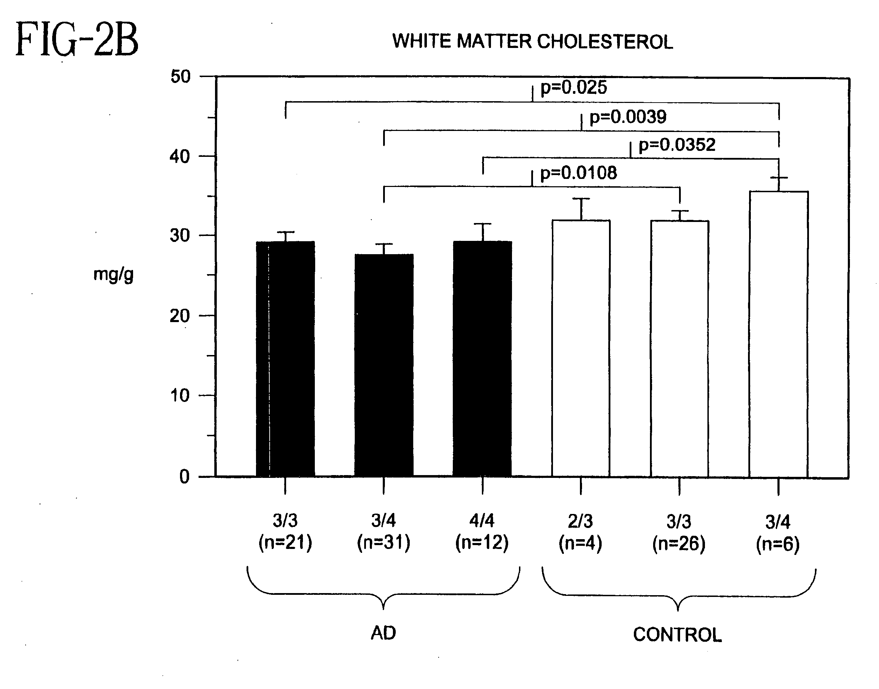

Examination of the lipid profiles of AD versus control subjects reveals a significant elevation in the amount of total cholesterol (TC), primarily in higher concentration of LDL in the AD cases (Table 1). This difference can be appreciated by its frequency distribution, segmented by decile, as shown in Figure 1. In controls subjects, 81 percent (29 of 36 subjects) had LDL cholesterol levels below the third decile (i.e., below 112 mg/dL), with all control subjects having LDL cholesterol below the fifth decile (i.e., below 163 mg/dL). In contrast, only 53 percent of the AD subjects fell below the third decile (36 of 68 subjects), while 21 percent (14 of 68 subjects) of these subjects had cholesterol above the fifth decile. Apolipoprotein B (ApoB), which is primarily associated with serum LDL, is also significantly elevated in AD (Table 1). Other lipids, such as VLDL-cholesterol, triglycerides (TG), ApoA-I, and ApoE, showed no significant differences between the AD and control groups (Table 1). In contrast, the levels of the HDL cholesterol, as well as the ratio of the HDL cholesterol to VLDL plus LDL cholesterol, were significantly higher in the control group than in the AD population (Table 1). As expected, the levels of Aβ N-40 and Aβ N-42 in brain were substantially higher in AD than those of control group (Table 1). When compared to the control group, the amount of brain white matter cholesterol in AD patients was less, as was the brain grey matter cholesterol, as shown in Figure 2.

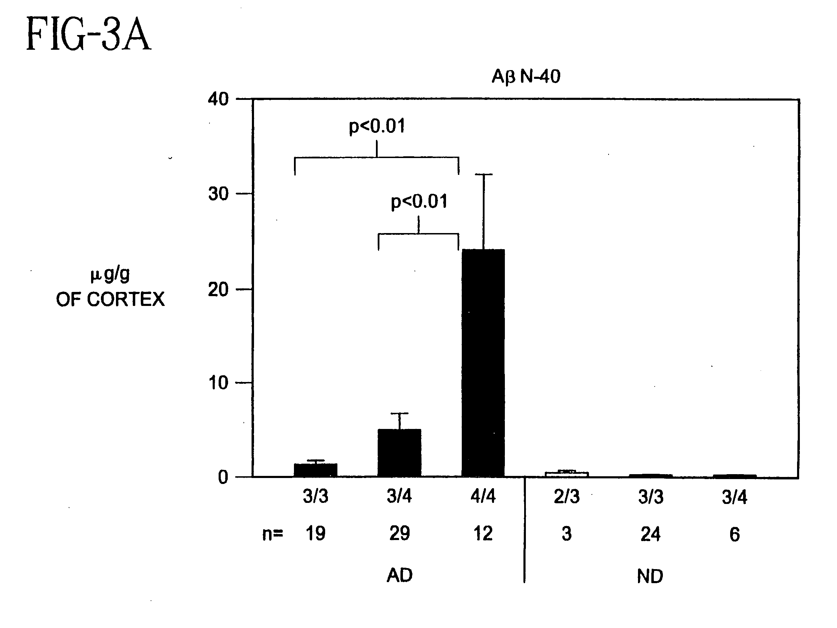

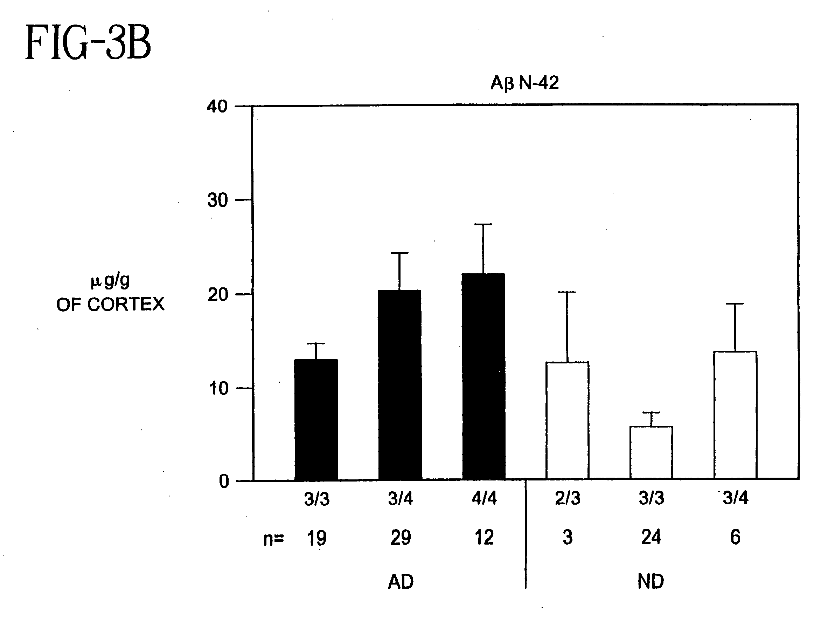

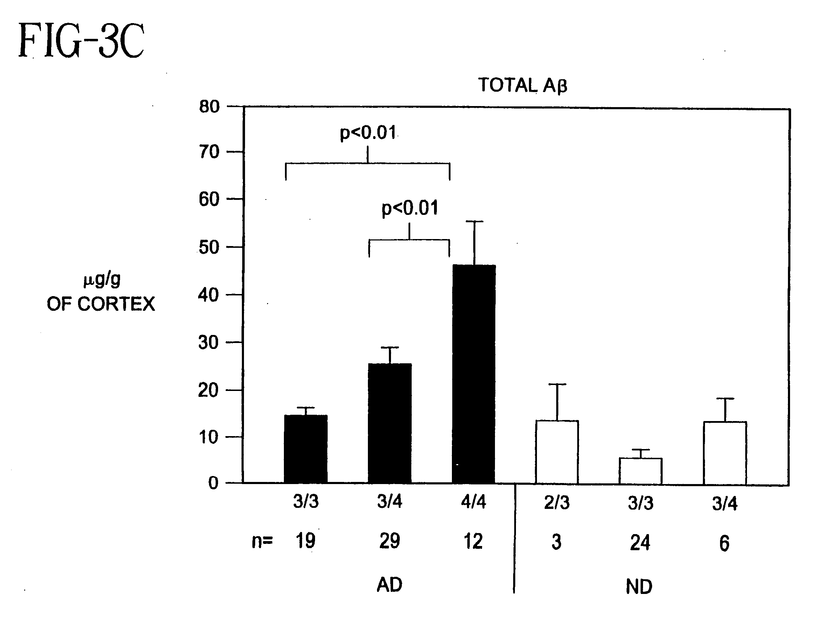

Large population studies show an effect of ApoE isoforms on serum total and LDL cholesterol levels. In our cohort, serum cholesterol levels were also increased in ApoE ε4 carriers; however, this elevation was not significant. The impact of ApoE genotype in this study is most evident on the amount of Aβ N-40 in AD brains (Figure 3A). The highest level of Aβ N-40 was found in AD patients homozygous for ApoE4, the amount being 20 times and 4 times greater than in those individuals with ApoE ε3/ε3 and ε3/ε4, respectively (Figure 3A). Any AD subjects carrying ApoE ε4 had approximately twice the quantity of Aβ N-42 when compared to those AD cases lacking the ApoE ε4 allele, as well as to all ApoE genotypes in the control group (Figure 3B). The sums of Aβ N-40 plus Aβ N-42 relative to each ApoE genotype are shown in Figure 3C. In AD subjects, the total Aβ linearly increased with the addition of one and two ApoE ε4 alleles (Figure 3C). In all cases, total Aβ was significantly higher in the AD subjects homozygous for ApoE4 than all other isoforms in either the AD or control cohorts (Figure 3C).

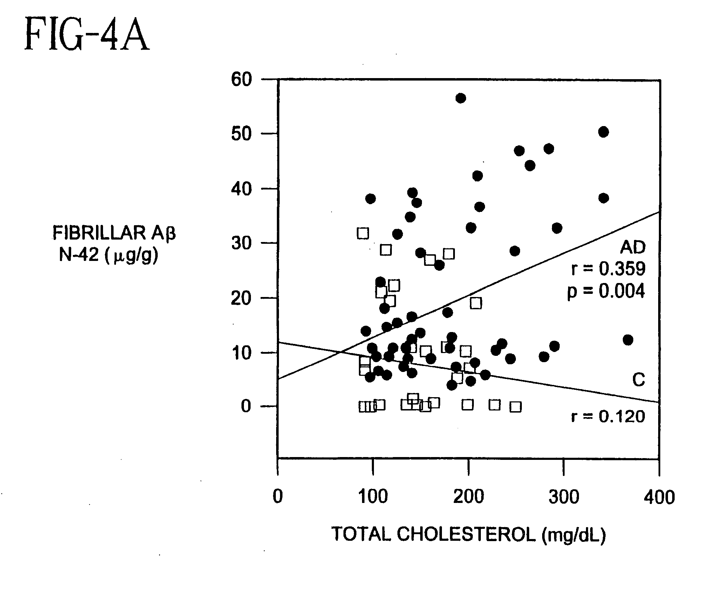

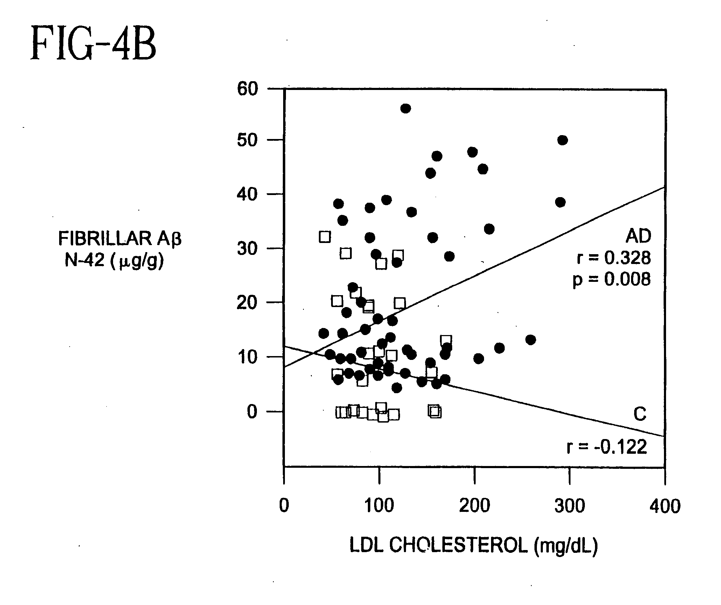

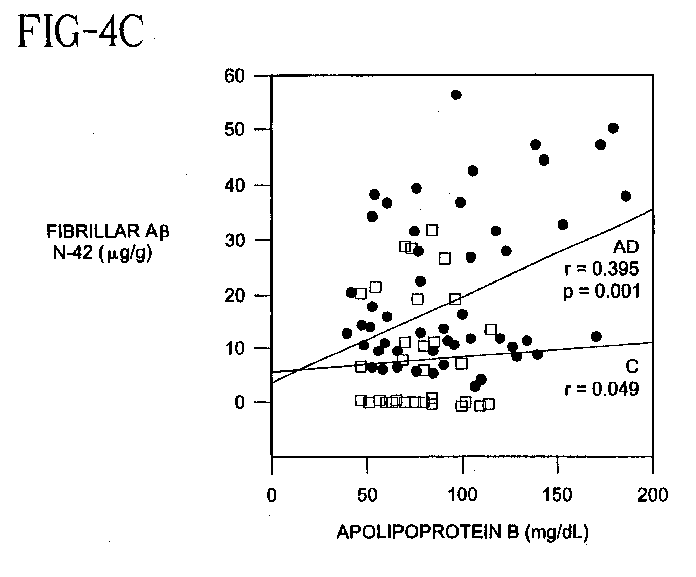

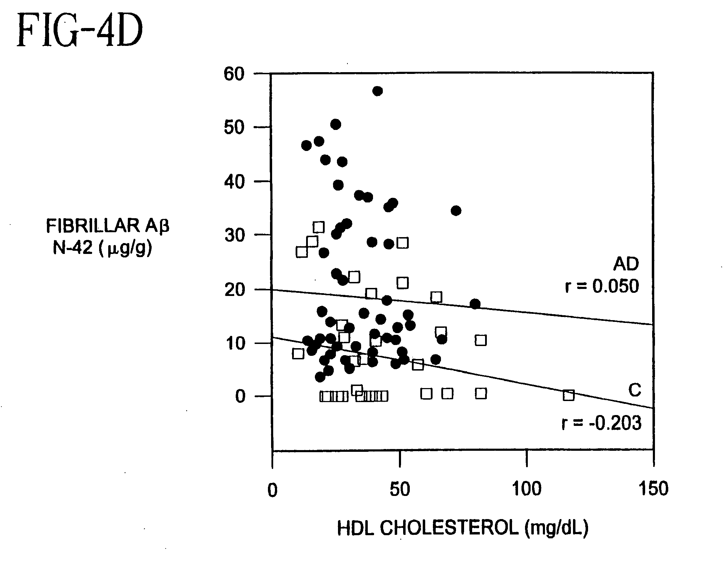

Significant associations between the levels of total serum cholesterol, LDL cholesterol and ApoB in AD subjects were seen with Aβ N-42 (Figures 4A-C), but not Aβ N-40 (data not shown). The strongest correlation occurred between ApoB and Aβ N-42 (Figure 4C), where the "r" value is the correlation factor, r = 1 being a perfect 1:1 correlation. These data clearly establish that those AD subjects with higher levels of total serum cholesterol, LDL cholesterol and ApoB are more likely to have higher levels of Aβ N-42. In control subjects (C), virtually no correlations were seen between these serum lipid parameters and Aβ N-42 levels (Figure 4A-C). The amounts of HDL also failed to show an association with Aβ N-42 in either control or AD brains (Figure 4D). These data establish that higher concentrations of total serum cholesterol leads to higher levels of β-amyloid peptide in AD brains.

The above study investigated whether factors associated with cardiovascular disease, such as high levels of serum total cholesterol, LDL cholesterol and low levels of HDL cholesterol, were associated with AD. The results establish that total serum and LDL cholesterol, as well as ApoB levels, are associated with increased deposition of Aβ N-42 in demented individuals with neuropathologically confirmed AD. The brain deposition of Aβ N-42 was significantly correlated with serum total and LDL cholesterol, and ApoB in the AD, but not in control subjects. There were also a disproportionate number of AD (47%) compared to control (18%) subjects with LDL cholesterol greater than 112 mg/dL (i.e., above the third decile for LDL cholesterol).

It is well-recognized that ApoE4 increases amyloid load in AD brain. The present data establish that the level of Aβ N-40 in AD brains appears governed almost exclusively by the presence of ApoE ε4. Aβ N-40 increases from 1.2 to 6.0 to 24.1 µg/g for 0, 1 and 2 copies of the ApoE ε4 allele, respectively. A similar but less dramatic trend is also observed for AβN-42. Immunological techniques have revealed an association between ApoE ε4 and higher concentrations of Aβ N-40 in AD cerebral cortex, and also between ApoE ε4 and vascular amyloid. Since most of Aβ N-40 is found in the cerebrovasculature, the foregoing data show that the presence of ApoE4 affects deposition of Aβ in blood vessels. The cerebrovascular amyloidosis observed in AD destroys the myocytes of small arteries and arterioles and obliterates the capillary network resulting in severe damage to cerebral blood flow. This compromise leads to neuronal damage through ischemia and hypoxia. Thus, ApoE ε4 may increase the risk of developing AD and accelerate its age of onset through indirect consequences on vessels in the brain.

Several lines of evidence have already suggested that cholesterol, or cholesterol metabolism, might influence susceptibility to AD. Two previous clinical studies showed that total serum or LDL cholesterol was elevated in patients with AD. In addition, individuals with ApoE ε4, a recognized risk factor for cardiovascular disease and AD, also tend to manifest hypercholesterolemia. Moreover, the incidence of AD appears to be higher in countries with high fat and caloric diets, and decreased in populations ingesting diets that decrease cardiovascular disease. Epidemiological investigations have further demonstrated that the risk for AD was greater in individuals with elevated cholesterol levels, and that the onset of AD occurred earlier in those individuals who were ApoE ε4 carriers with high serum cholesterol.

**Table 1.**

| Comparison Between AD and Control Subjects With Respect to Serum Lipids and Brain Tissue Aβ N-40 and Aβ N-42 | | | |
|---|---|---|---|
| | AD (n = 64) | Control (n = 36) | P value* |
| age (years) | 81.6 ± 0.9 | 78.7 ± 1.3 | 0.054 |
| TC (mg/dL) | 176.0 ± 8.2 | 152.8 ± 7.1 | 0.061 |
| VLDL-C (mg/dL) | 18.6 ± 2.0 | 17.0 ± 2.0 | 0.619 |
| LDL-C (mg/dL) | 124.0 ± 7.0 | 95.5 ± 5.0 | **0.006** |
| HDL-C (mg/dL) | 35.0 ± 1.8 | 42.3 ± 3.7 | **0.040** |
| HDL-C/(VLDL-C+LDL-C) | 0.31 ± 0.03 | 0.41 ± 0.04 | **0.048** |
| TG (mg/dL) | 225.3 ± 12.6 | 201.4 ± 16.0 | 0.249 |
| ApoA-I (mg/dL) | 100.0 ± 3.3 | 108.2 ± 5.1 | 0.162 |
| ApoB (mg/dL) | 91.8 ± 4.4 | 76.6 ± 3.1 | **0.018** |
| ApoE (mg/dL) | 4.8 ± 0.3 | 5.0 ± 0.4 | 0.753 |
| Aβ N-40 (µg/g) | 7.47 ± 2.05 | 1.11 ± 0.56 | **0.024** |
| Aβ N-42 (µg/g) | 18.2 ± 1.7 | 7.87 ± 1.68 | **<0.001** |
| Aβ Total (µg/g) | 25.7 ± 2.8 | 9.0 ± 1. 9 | **<0.001** |
| TC = Total serum cholesterol; VLDL-C = Very low density lipoprotein cholesterol; LDL-C = Low density lipoprotein cholesterol; HDL-C = High density lipoprotein cholesterol; TG = Triglycerides. | | | |

| | | | |
|---|---|---|---|
| * Two-tailed Student T-test probability | | | |

### EXAMPLE 2

This experiment was designed to determine the ability of lipid regulating agents to alter the production of β-amyloid peptide (Aβ) in cultured cells, and their consequent activity in preventing and treating Alzheimer's Disease.

Chinese hamster ovary (CHO) cells were stably transfected with a construct to enable the overexpression of the human β-amyloid precursor protein (βAPP) gene to cause increased production of Aβ. The measurement of Aβ synthesized by these βAPP-CHO cells was done using a standard sandwich ELISA assay, employing well-characterized antibodies to the N-terminus (6E10) and middle (4G8) of Aβ. This assay is routinely used to measure Aβ in tissues, body fluids, and cell culture media.

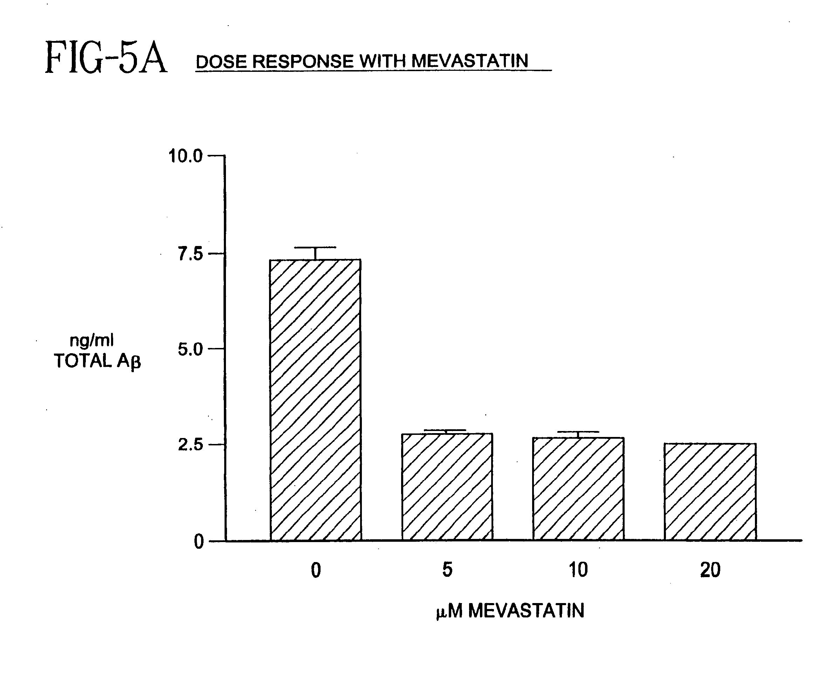

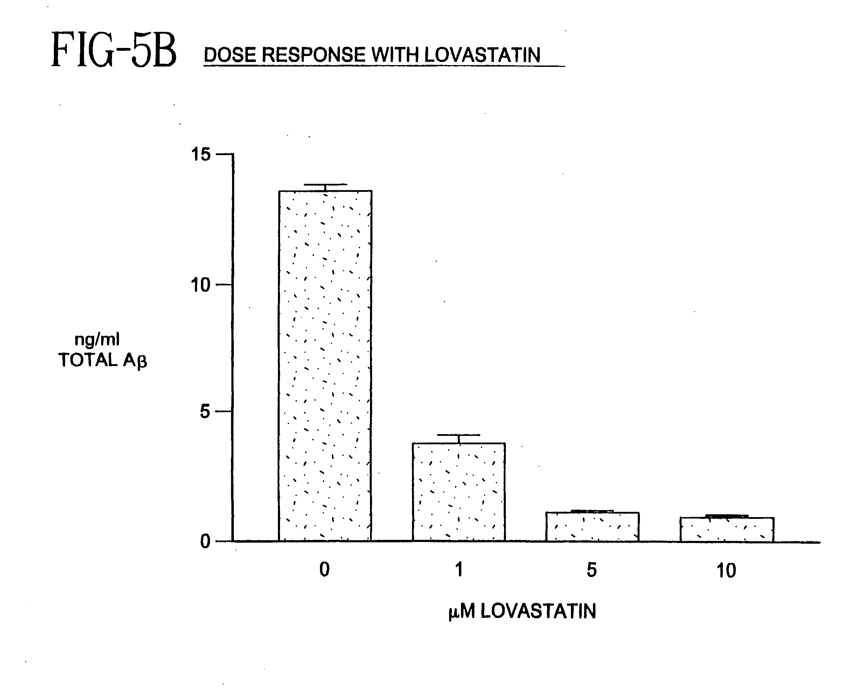

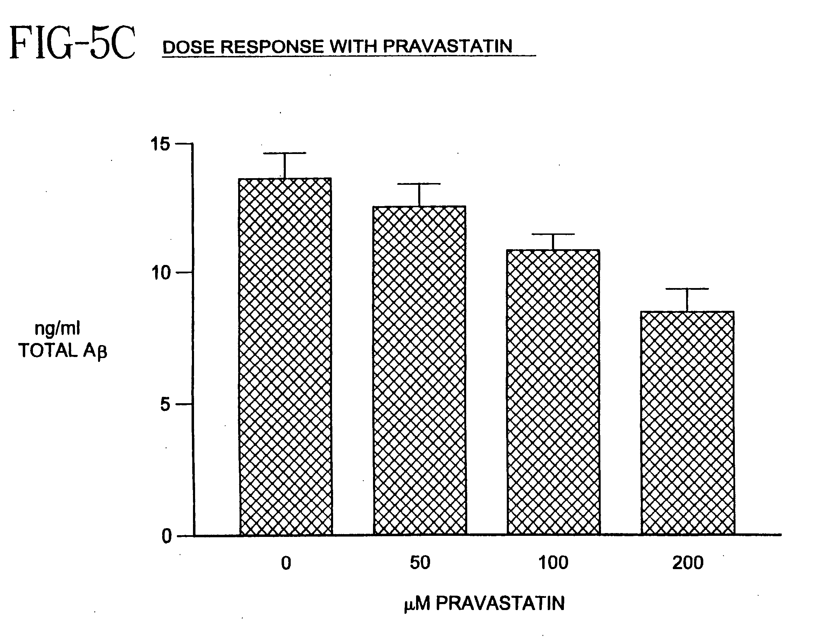

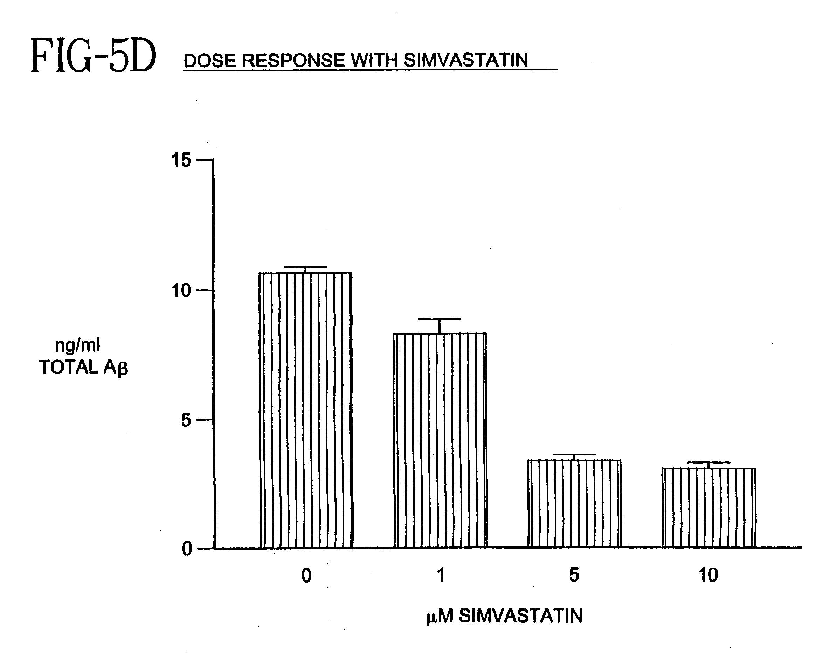

Cultures of βAPP-CHO cells were grown to near confluency, and then the test compounds were added at various dose concentrations to the cell medium. Figure 5 shows the dramatic reduction in Aβ caused by several statins. Mevastatin, lovastatin, and simvastatin all caused a dramatic dose-dependent reduction in Aβ. Pravastatin caused a dose-dependent reduction in Aβ as well, albeit somewhat less pronounced.

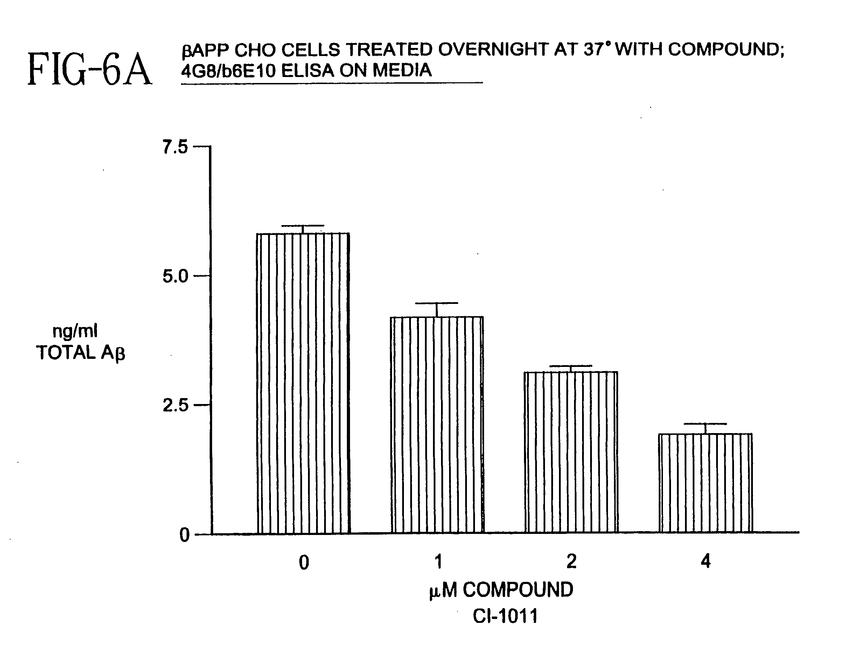

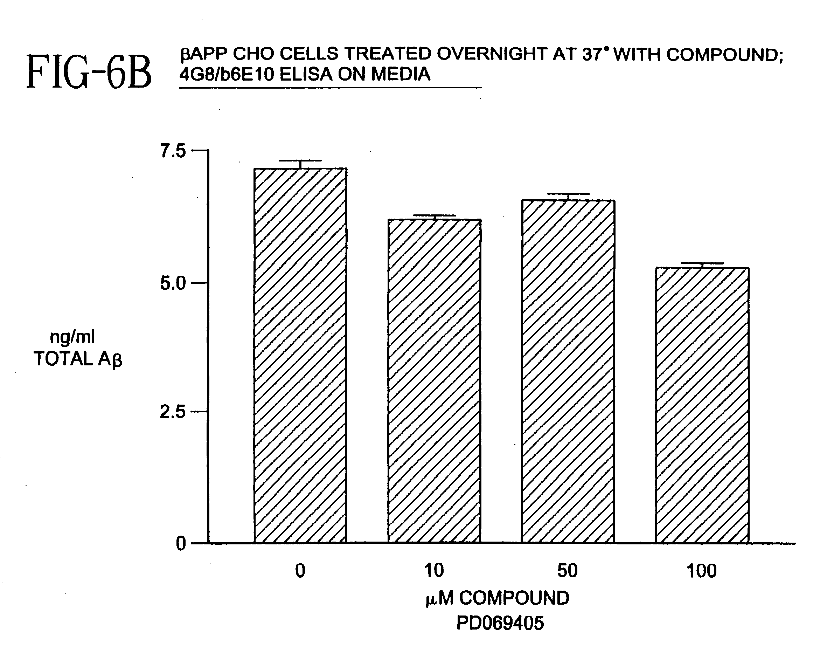

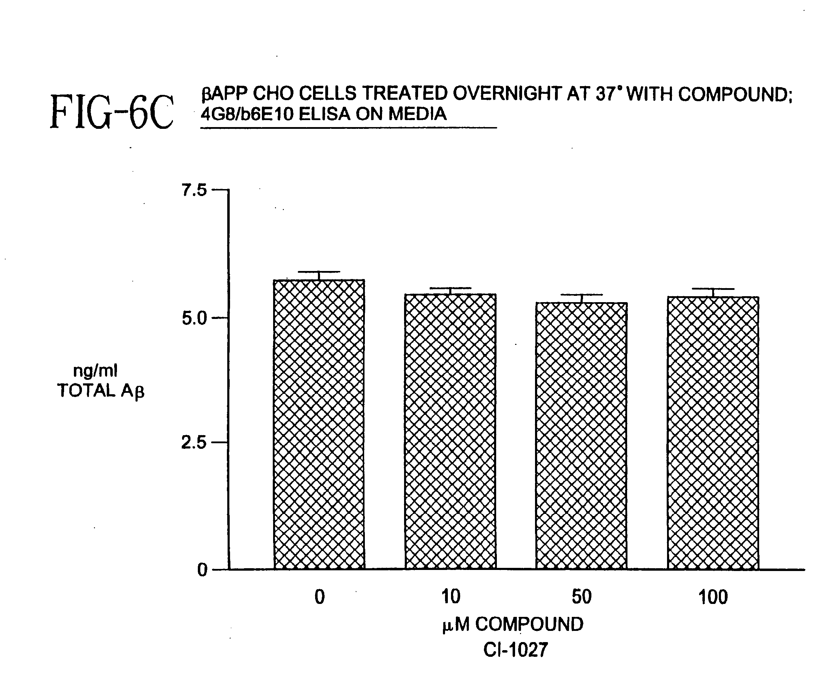

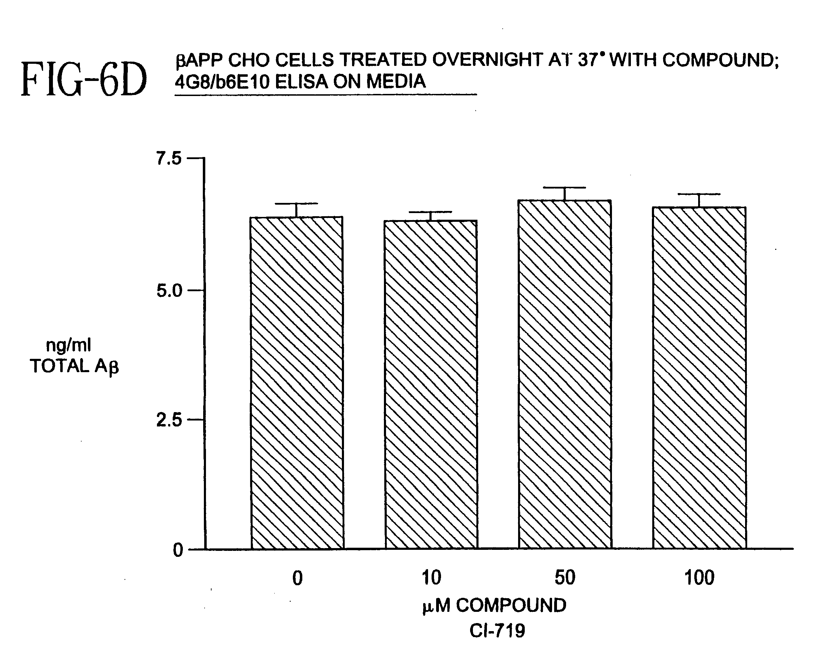

Several other lipid regulating agents were evaluated in the βAPP-CHO cells. Avasimibe (CI-1011) caused a substantial dose-dependent reduction in Aβ, as shown in Figure 6. PD 69405, CI-1027, and CI-719 caused only moderate changes at the concentrations tested.

### EXAMPLE 3

The following experiment established that lipid regulating agents cause a reduction in insoluble fibrillar Aβ N-42 in the brains of animals.

Mice aged 24 months were fed a high fat (15%) high cholesterol (1.25%) diet containing 0.5% cholic acid (High Fat) or regular rodent chow (chow) for 4 weeks. During the last 2 weeks of the study, two groups of mice were given 10 mg/kg simvastatin daily by oral gavage. Mice were then sacrificed by anesthetic overdose perfused with cold 0.9% saline via heart puncture. The saline rinsed brain was then removed from the skull and frozen over dry ice. The brain samples were stored at -80°C until assayed for Aβ N-40 and Aβ N-42.

On the day of assay, brains were thawed and the hippocampus and cortex were dissected from the rest of the brain. These samples were dounce homogenized in tris-buffered saline (TBS) containing protease inhibitor cocktail (PIC) and 0.5 mM ethylene diamine tetraacetic acid (EDTA). The samples were centrifuged at 100,000XG for 1 hour. The supernatants were drawn off, and the remaining pellet was treated with 0.2% diethylamine buffer in 50 mM saline. The pellet was re-suspended in diethylamine (DEA) by probe sonication, and the samples were centrifuged again at 100,000XG for 1 hour. The DEA extracted supernatant samples were drawn off and neutralized to pH 8.0 by the addition of 2 M tris-HCl buffer. The amount of Aβ N-40 and Aβ N-42 were measured in these samples by ELISA. In addition, a protein assay was run on each sample so that variations in sample size could be normalized by protein content. Thus, Aβ values are expressed in ng/mg protein.

Table 7 shows that the lipid regulating agent simvastatin (S) caused a substantial reduction in Aβ N-42 in all animals, compared to non-treated controls (C). The animals having the High Fat diet exhibited slightly less inhibition of Aβ N-42 than the Chow fed animals. The compound had only marginal effect on Aβ N-40.

## Claims

1. Use of a plasma-triglyceride level-lowering agent for the preparation of a pharmaceutical composition for treating Alzheimer's Disease wherein the agent is an inhibitor of acylcoenzyme A (ACAT inhibitors).

2. Use of claim 1 wherein the ACAT inhibitor is a compound of formula 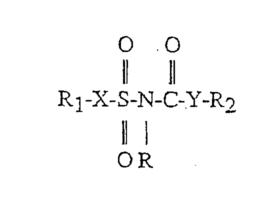 wherein X and Y are O, S, or (CR'R")ₙ, n is 1 to 4, R is hydrogen, alkyl or benzyl, R₁ and R₂ is aryl, cycloalkyl, R' and R" are each independently hydrogen, alkyl, alkoxy, halogen, hydroxy, acycloxy, cycloalkyl, phenyl optionally substituted or R'and R" together form a spirocycloalkyl or carbonyl.

3. Use of claim 1 to 2 wherein the ACAT inhibitor is 2,6-bis (1-methylethyl) phenyl [[2,4,6-tris(1-methylethyl)phenyl]acetyl]sulfamate.

4. Use according to claim 1 to 3 wherein the agent is co-administered with a plasma cholesterol level-lowering agent.

5. Use according to claim 4 wherein the plasma cholesterol level-lowering agent is selected from the group consisting of statins, bile acid sequestrants, and agents that block intestinal cholesterol absorption.

6. Use according to claim 5 wherein the plasma cholesterol level-lowering agent is mevastatin, simvastatin, pravastatin, atorvastatin, cenvastatin, fluvastatin, lovastatin, cholestyramine and colestipol.

7. Use of plasma-triglyceride level-lowering agent for the preparation of a pharmaceutical composition for preventing the onset Alzheimer's Disease wherein the agent is an inhibitor of acylcoenzyme A (ACAT inhibitors).

8. Use of claim 7 wherein the ACAT inhibitor is a compound of formula 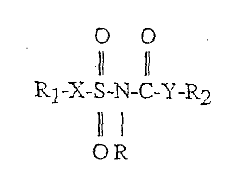 wherein X and Y are O, S, or (CR'R")ₙ, n is 1 to 4, R is hydrogen, alkyl or benzyl, R1 and R2 is aryl, cycloalkyl, R' and R" are each independently hydrogen, alkyl, alkoxy, halogen, hydroxy, acycloxy, cycloalkyl, phenyl optionally substituted or R'and R" together form a spirocycloalkyl or carbonyl.

9. Use of claim 7 to 8 wherein the ACAT inhibitor is 2,6-bis (1-methylethyl) phenyl [[2,4,6-tris(1-methylethyl)phenyl]acetyl]sulfamate.

10. Use according to claim 7 to 9 wherein the agent is coadministered with a plasma cholesterol level lowering agent.

11. Use according to claim 9 wherein the plasma cholesterol level-lowering agent is selected from the group consisting of statins, bile acid sequestrants, and agents that block intestinal cholesterol absorption.

12. Use according to claim 11 wherein the plasma cholesterol level-lowering agent is mevastatin, simvastatin, pravastatin, atorvastatin, cenvastatin, fluvastatin, lovastatin, cholestyramine and colestipol.
